# EUROPEAN PATENT SPECIFICATION

(11) **EP 2 128 172 B1**
(45) Date of publication and mention of the grant of the patent: **11.09.2013**
(21) Application number: 08700789.4
(22) Date of filing: 17.01.2008
(51) Int. Cl.: C12N 15/12, C07K 14/47, C07K 1/14, C07K 1/18, C07K 1/30

(54) **PREPARATION PROCESS OF RECOMBINANT HUMAN P43 PROTEIN**
VERFAHREN ZUR HERSTELLUNG VON REKOMBINANTEM HUMANEM P43-PROTEIN
PROCÉDÉ DE PRÉPARATION DE LA PROTÉINE HUMAINE RECOMBINANTE P43

(30) Priority: 18.01.2007 CN 200710036577
(43) Date of publication of application: 02.12.2009
(73) Proprietor: Sine Pharmaceutical Laboratories, Shanghai 201206 (CN)
(72) Inventor: LIU, Datao, Shanghai 201206 (CN); TAN, Xiaoding, Shanghai 201206 (CN); HU, Linda, Shanghai 201206 (CN); WANG, Yan, Shanghai 201206 (CN); ZHU, Xi, Shanghai 201206 (CN)
(74) Representative: Spencer, Matthew Peter
(86) International application number: PCT/CN2008/070132
(87) International publication number: WO 2008/089690

(56) References cited:
- WO-A1-01/95927
- CN-A- 1 341 727
- US-B2- 6 734 168
- SHALAK VYACHESLAV ET AL: "Characterization of p43(ARF), a derivative of the p43 component of multiaminoacyl-tRNA synthetase complex released during apoptosis." THE JOURNAL OF BIOLOGICAL CHEMISTRY 13 APR 2007 LNKD- PUBMED:17303557, vol. 282, no. 15, 13 April 2007 (2007-04-13), pages 10935-10943, XP002591679 ISSN: 0021-9258
- LEE S W ET AL: "Aminoacyl-tRNA synthetase complexes: beyond translation" JOURNAL OF CELL SCIENCE, CAMBRIDGE UNIVERSITY PRESS, LONDON, GB LNKD- DOI:10.1242/JCS.01342, vol. 117, no. 17, 1 August 2004 (2004-08-01), pages 3725-3734, XP008095224 ISSN: 0021-9533
- YI JAE-SUNG ET AL: "Aminoacyl-tRNA synthetase-interacting multi-functional protein, p43, is imported to endothelial cells via lipid rafts." JOURNAL OF CELLULAR BIOCHEMISTRY 15 DEC 2005 LNKD- PUBMED:16167337, vol. 96, no. 6, 15 December 2005 (2005-12-15), pages 1286-1295, XP002591680 ISSN: 0730-2312
- HAN ET AL: "Structural separation of different extracellular activities in aminoacyl-tRNA synthetase-interacting multi-functional protein, p43/AIMP1" BIOCHEMICAL AND BIOPHYSICAL RESEARCH COMMUNICATIONS, ACADEMIC PRESS INC. ORLANDO, FL, US LNKD- DOI:10.1016/J.BBRC.2006.01.117, vol. 342, no. 1, 31 March 2006 (2006-03-31), pages 113-118, XP005291574 ISSN: 0006-291X
- WEI J. ET AL.: 'Role of Hydrophobic Interaction Chromatography in Purification of rProEMAP II/P43 Protein Expressed in E. coli' CHINESE JOURNAL BIOLOGICALS vol. 19, no. 1, January 2006, pages 73 - 76, XP008136064
- QUEVILLON S. ET AL.: 'The p43 component of the mammalian multi-synthetase complex is likely to be the precursor of the endothelial monocyte-activating polypeptide II cytokine' J. BIOL. CHEM. vol. 272, no. 51, 19 December 1997, pages 32573 - 32579, XP008116659

## Description

### Technical Field

The present invention relates to a process for preparing recombinant human protein, in particular to a process for preparing recombinant human p43 protein.

### Background Art

Malignant tumor is very harmful to health of human being. The methods for treating malignant tumor in the medical field mainly comprise killing cancer cell via surgery, chemotherapy and teletherapy, and the like. These methods may kill normal cell when killing cancer cell, and they may readily make the cancer cell producing drug resistance. Therefore, people has all the while found a method that could specifically kill cancer cell without damaging normal cell, and at the same time did not make the cancer cell producing drug resistance. Currently, there emerge in endlessly a lot of new drugs for treating tumor. Recently, people gradually focuses on the methods for treating malignant tumor by genetic engineering drug, with development of molecular biology gene engineering. Conventional chemotherapy for treating tumor uses drugs having cytotoxicity which may damage not only cancer cells but also normal cells, thereby weakening human body condition. So, many patients of advanced cancer finally die because they are unable to endure the treatment. Malignant tumors have one common feature, i.e., cells in a local area greatly and un-controllably proliferate. In order to maintain this cell proliferation, it needs to provide much nutrition. When forming tumor, many blood vessels for supplying nutrition components are formed within and around the tumor. If the "channels" for supplying are cut to interrupt the nutrition components supply for the tumor, the tumor will die due to depletion.

A method of inhibiting neovascularization is recently developed for completely treating cancer. The method of inhibiting neovascularization may stop the formation of the blood vessels in tumor mainly through inhibiting the growth of vascular endothelial cell that fast and abnormally proliferate, thereby specially inhibiting the growth of proliferating vascular endothelial cells in tumor. It atrophies capillary vessel of tumor, so the nutrition supply of tumor is cut and the cancer cell is dead. The object of treating cancer is thus achieved.

In recent 10 years, a plurality of potential inhibitors for neovascularization have been found, including human endostatin. It was firstly reported in Cell, January 1997 about Endostatin by Folkman from Medical College, Harvard, finding that two drugs (Angiostatin and Endostatin) may remove tumor in rat without recurrence. These two drugs can treat cancer by stopping the growth of blood vessels which supply nutrition components for tumor. This made a big splash at that time. Endostatin was rapidly studied in the field of cancer treatment. In the beginning of 1999, at that time the clinical studies were not completed, FDA specifically admitted clinical trial for using a mixed gene engineering injection of Angiostatin and Endostatin for treating 30 patients with advanced cancer which were non-responded to the other drugs. Experts in American Society of Clinical Oncology believe that these drugs may be successful. Now, Endostatin has passed the first clinical trial and it entered into the second clinical trial. However, since Endostatin has poor solubility, resulting in high cost of production. Meanwhile, it is used only in aqueous injection dosage form, which greatly limits its applications.

p43 protein is an accessory factor of tRNA synthetase of mammals, which on one hand adjusts directly the formation of capillary vessel from endothelial cells, and on the other hand inhibits the formation of blood vessel of tumor by changing microenvironment. The activity of p43 in inhibiting formation of blood vessel and growth of tumor have been proved by in vitro tests and animal tests. Human gene recombinant p43 protein shows the potential of being developed into a new drug for treating cancer. It may be effective in treating a plurality of primary and metastatic solid tumors and may be used along with chemotherapy and teletherapy.

p43 protein is a member of human aminoacyl-tRNA synthetase and a precursor of endothelial monocyte active peptide, which have been found in 1997 by Sophie Q, et al. p43 protein is a single-chain protein, comprising 312 amino acids totally and its secondary structure includes 11β - sheets. Imagene Company in South Korea have thoroughly studied the structure and activity of human p43 protein, finding that p43 protein may inhibit the growth of vascular endothelial cell that proliferate fast and abnormally and the growth of neogenic vessels in hen'egg test-chorioallantoic membrane, which means that p43 protein may have a potential of treating cancer.

In order to develop p43 protein as a drug, it is necessary to express a plurality of p43 protein by genetic engineering process and purify same, thereby obtaining a given amount of high purity protein. Recently, conventional molecular biology process is used to clone this protein into prokaryote expression carrier. High expression can be obtained in *Escherichia coli.* However, there is a problem in its purification, mainly because it is difficult to separate p43 protein from nucleic acid. As a result, purification yield of the protein decreases greatly.

P43 protein can easily combine with nucleic acid. Under the general condition, and in the homogenate of genetic engineering bacteria, many p43 proteins are combined with the nucleic acid obtained from disrupted cell, such that the charges carried by p43 protein are nonuniform and the molecular weights of p43 protein are also nonuniform. Therefore, it is very difficult to purify the protein, resulting in the great decrease of yield of the protein.

Therefore, there is a need for providing a process of effectively purifying p43 protein from genetic engineering bacteria containing p43 protein, with high yield.

Wei J. et al. (2006) Chinese Journal Biologicals, vol. 19, no. 1, 73-76 relates to purification of rProEMAP II / P43 protein.

### Summary of the Invention

The object of the present invention is to provide a process of effectively purifying p43 protein from genetic engineering bacteria containing p43 protein gene, with high yield.

The present invention provides a process of producing recombinant p43 protein from genetic engineering *Escherichia coli* containing p43 protein gene, comprising the following steps:
(1) suspending genetic engineering *Escherichia coli* containing p43 protein gene by buffer solution to obtain a suspension;
(2) disrupting and centrifuging the suspension obtained in step (1) to obtain a supernatant;
(3) salting out the supernatant obtained in step (2) with ammonium sulfate solution to obtain a precipitate, and dissolving the obtained precipitate with a buffer solution to obtain a solution;
(4) treating the solution obtained in step (3) by passing it through the SP Sepharose^{™} Fast Flow column;
(5) eluting the SP Sepharose^{™} Fast Flow column with a buffer solution to obtain p43 protein solution.

In one preferred embodiment according to the present invention, the buffer solution is one or more solutions selected from the group consisting of trimethylolaminomethane hydrochloride and phosphate solutions.

In one preferred embodiment according to the present invention, the buffer solution has a pH of from 6.5 to 9.0.

In one preferred embodiment according to the present invention, the buffer solution has a pH of from 7 to 8.5.

In one preferred embodiment according to the present invention, the buffer solution has a pH of from 7.0 to 8.0.

P43 protein can easily combine with nucleic acid. Under the general condition, and in the homogenate of genetic engineering bacteria, many p43 proteins are combined with the nucleic acid obtained from disrupted cell, such that the charges carried by p43 protein are non-uniform and the molecular weights of p43 protein are also non-uniform. Therefore, it is very difficult to purify the protein, resulting in the great decrease of yield of the protein. The present invention can effectively separate the target protein from nucleic acid by optimizing the purification process. As a result, the purification efficiency and yield can be greatly increased.

### Brief Description of Figures

Figure 1 describes an expression result of electrophoresis measure.
Figure 2 describes the SDS-PAGE electrophoresis of the protein elution peak and the through-peak after passing through the SP column.

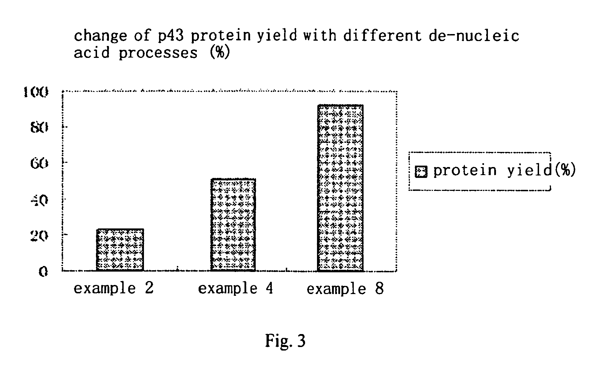

Figure 3 describes the change of p43 protein yield with separation processes for removing nucleic acid.

### Detailed Description of the Invention

The present invention provides a process of producing recombinant p43 protein from genetic engineering *Escherichia coli* containing p43 protein gene, comprising the following steps:
(1) suspending genetic engineering *Escherichia coli* containing p43 protein gene by buffer solution to obtain a suspension;
(2) disrupting and centrifuging the suspension obtained in step (1) to obtain a supernatant;
(3) salting out the supernatant obtained in step (2) with ammonium sulfate solution to obtain a precipitate, and dissolving the obtained precipitate with a buffer solution to obtain a solution;
(4) treating the solution obtained in step (3) by passing it through the SP Sepharose™ Fast Flow column;
(5) eluting the SP Sepharose™ Fast Flow column with a buffer solution to obtain p43 protein solution.

It is conventional in the art to obtain genetic engineering *Escherichia coli* containing p43 protein gene. See "*molecular cloning*" (2^{nd} Edition). In one embodiment according to the present invention, genetic engineering *Escherichia coli* containing p43 protein gene is prepared by the following process: synthesizing full length gene according to DNA sequence of known human p43 protein gene; introducing an Nco I restriction site into the 5'-end of the gene sequence; introducing an Xho I restriction site into the 3'-end; double cleaving by Nco I and *Xho*I I enzymes, and then inserting the cleaved product into an pET28a plasmid having been cleaved by said two enzymes; forming an pET28a-43 plasmid containing human p43 protein gene; confirming the gene sequence by DNA sequencing analysis; using *Escherichia coli* BL21-DE3 as a host bacteria to transform it with the pET28a-43 plasmid and expressing; obtaining genetic engineering *Escherichia coli* containing p43 protein gene having stably high expression by repeating screen and expression analysis.

The buffer solution in step (1) according to the present invention is conventional in the art. A skilled person in the art can deduce, according to the disclosure of the present invention as well as the prior art, which buffer solution can be used in the present invention. In one preferred embodiment according to the present invention, the buffer solution is one or more solutions selected from the group consisting of trimethylolaminomethane hydrochloride and phosphate solutions.

The pH of the buffer solution according to the present invention is conventional in the art. A skilled person in the art can determine, according to the disclosure of the present invention as well as the prior art, the specific range of pH value of the buffer solution. In one preferred embodiment according to the present invention, the buffer solution has a pH of 6.5-9.0, preferably 7-8.5, more preferably 7.0-8.0.

The disrupting process and centrifugation process in step (2) according to the present invention are conventional in the art. A skilled person in the art can deduce, according to the disclosure of the present invention as well as the prior art, which processes may be used in the present invention. In one preferred embodiment according to the present invention, the disrupting process comprises the step of disrupting thallus by high pressure homogenizing at a condition of 1000 atm and one cycle. In another preferred embodiment according to the present invention, the centrifugation process is carried out at 5000-20000 rpm, preferably at 12000-15000 rpm.

The ammonium sulfate solution in step (3) according to the present invention is conventional in the art. A skilled person in the art can deduce, according to the disclosure of the present invention as well as the prior art, which ammonium sulfate solution can be used in the present invention. In one preferred embodiment according to the present invention, the ammonium sulfate solution is 60% by weight of aqueous solution.

The buffer solution in step (5) according to the present invention is conventional in the art. A skilled person in the art can deduce, according to the disclosure of the present invention as well as the prior art, which buffer solution can be used in the present invention. In one preferred embodiment according to the present invention, the buffer solution in step (5) is the same as the buffer solution in step (1), or the buffer solution in step (5) may comprise other components such as sodium chloride in addition to those in the buffer solution in step (1).

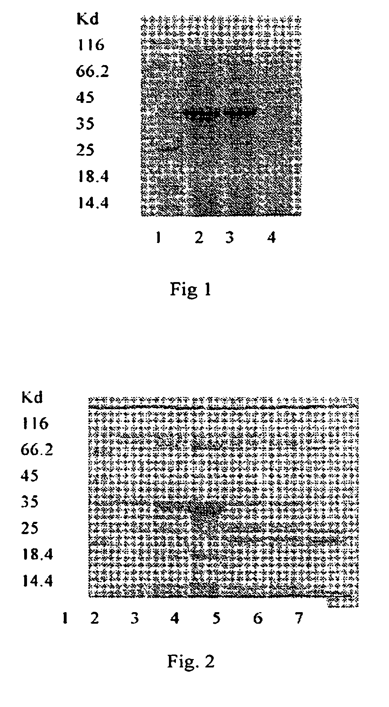

In figure 1, reference numbers 1-4 refer to the followings:
1. standard protein molecular weight Marker
2. high expression strain
3. high expression strain
4. control before inducement

In figure 2, reference numbers 1-7 refer to the followings:
1. standard protein molecular weight Marker
2. the elution peak from the sp column after separation of protein from nucleic acid in example 2
3. the elution peak from the sp column after separation of protein from nucleic acid in example 4
4. the elution peak from the sp column after separation of protein from nucleic acid according to the optimized method of the present invention in example 8
5. the through-peak from the sp column after separation of protein from nucleic acid in example 4
6. the through-peak from the sp column after separation of protein from nucleic acid in example 2
7. the through-peak from the sp column after separation of protein from nucleic acid according to the optimized method of the present invention in example 8

### Examples

### Example 1: Construction of genetic engineering bacteria containing human p43 protein

### 1. Construction of expression plasmid containing a gene sequence for human p43 protein

Primer A: ACACCATGGCAAATAATGATGCTGTTC and primer B: ACTCGAGTTATTTGATTCCACTGTTGCTC were designed. PCR amplification was carried out by the pUC-18 plasmid containing fully-synthesized gene sequence for p43 protein as a template via a standard method, which include 30 cycles of denaturalizing at 94°C for one (1) minutes; annealing at 55°C for 40 seconds; extending at 72°C for 1.5 minutes, thus obtaining a segment containing the gene for p43 protein, whose 5'-end and 3'-end have Nco I and Xho I restriction sites, respectively. The pET28a plasmid after extraction and purification was cleaved by both Nco I and Xho I (available from takara company). The reaction system had a total volume of 20 µl as follows:

| | |
|---|---|
| pET28a | 15 µ l |
| Nco I | 0.5 µ l |
| Xho I | 0.5 µ l |
| 10 × buffer K | 2 µ l |
| BSA(bovine serum albumin) | 2 µ l |

The reaction temperature was 37°C and the time was 1.5 hours.

The gene segment was doubly cleaved by the same method. The reaction system had a total volume of 20µl as follows:

| | |
|---|---|
| PCR product | 10 µ l |
| Nco I | 0.5 µ l |
| Xho I | 0.5 µ l |
| 10 × buffer K | 2 µ l |
| BSA | 2 µ l |
| ddH₂O | 5 µ l |

The reaction temperature was 37°C and the reaction time was 1.5 hours.

The product after cleavage and purification was ligated. The reaction system had a total volume of 15 µl as follows:

| | |
|---|---|
| pET28a/_{Nco I+Xho I} | 1 µ l |
| p43/_{Nco I+Xho I} | 5 µ l |
| 10 × buffer H | 1.5 µ l |
| ligase | 0.5 µ l |
| ddH₂O | 7 µ l |

The reaction temperature was 16°C and the time was 24 hours. All the reagents in the above steps are available from DALIAN BAO BIOLOGY COMPANY, Liaoning, China.

The ligated product was used to transform the *Escherichia coli* TOP10 (available from SHANGH PUFEI BIOLOGY COMPANY). By identifying by the above PCR and double enzyme cleavage processes, positive clones were selected. The plasmid was extracted after culturing the positive clones to obtain expression vector pET28a-43 containing the gene sequence for human p43 protein.

### 2. Construction of engineering bacteria

*Escherichia coli* BL21 (DE3) (available from SHANGH PUFEI BIOLOGY COMPANY) was stored in the lab of the applicant. A single colony was selected from a newly-cultured LB plate and transferred into 2ml of LB, and it was shaked at 37°C over night. 1% of the single colony was inoculated into 50ml of LB culture medium. At the same conditions, the colony was cultured until OD600=0.4. The culture was treated at an ice bath for 10 minutes and centrifugated at 4°C and 3000rpm for 10 minutes to recover the thallus. The thallus was re-suspended by 10 ml of pre-cooled 0.1M calcium chloride solution on ice for 30 minutes. Cells were recovered by centrifugation at 4°C and 3000rpm for 10 minutes. The competent cells were prepared by re-suspending the cells with 2 ml of pre-cooled 0.1 M calcium chloride solution. About 0.5-2 µg of expression plasmid pET28a-43 was added into 200 µl of competent cells in ice bath for 30 minutes, thermally shocking at 42°C for 90 seconds and then adding into 1.5 ml of LB culture medium at 37°C in warm bath for 1 hour. The product was coated onto an LB plate containing 30 µg/ml kanamycin and cultured in an incubator at 37°C over night. The grown clones were selected to be inoculated to 2 ml of LB culture medium at 37°C over night. The cultured clones were inoculated to 3 ml of LB in a ratio of 1:50 and then cultured at 37 °C until OD600=0.4-0.6. Then IPTG (isopropyl-b-D-thiogalactoside) was added to a final concentration of 0.4-0.8 mmol. The product was induced for expression for 6 hours. Samples were obtained for SDS-PAGE electrophoretic analysis every two hours. The samples were stained by Commassie Blue and scanned for analyzing the amount of the recombinant protein. High expression clones were obtained (See figure 1).
3. Obtainment of bacterial sludge: Fermentation medium was the conventional M9 culture medium. The inoculation amount was about 2% to about 5%. The culture was started at 30°C and pH of 7.0. During culture, the temperature was controlled at 30°C and DO was controlled above 25% by increasing rotate speed. The fermentation solution was maintained at pH of about 7.0 with 4M NaOH. In the middle-final stage of fermentation, glucose was added intermittently. And at the same time YE. was also added. When OD=14-15, IPTG (isopropyl-b-D-thiogalactoside) was added to induce expression. The final concentration of IPTG was 1 mmol/L, and the induction time was 4 hours. OD600 was determined. When OD600=about 40, the product was added into a jar. (Culturing condition) The bacterial solution was centrifugated by tube-type centrifuge (available from SHANGHAI CENTRIFUGE INSTITUTE) at 10000rpm to obtain bacterial sludge. The bacterial sludge was stored at -70°C.

### Example 2

The bacterial sludge of *Escherichia coli* expressing p43 protein obtained in example 1 was dissolved in a solution of 50mM of trimethylolaminomethane hydrochloride having a pH of 7.0, 2mM of mercaptoethanol and 5mM of EDTA at a ratio of 1:10. The cells were disrupted by high pressure homogenizing method. The disruption solution was centrifugated at 15000rpm and 4°C and the centrifugated deposit was removed. This solution was used as a loading solution, which was passed through an SP Sepharose™ Fast Flow (GE Company) column. After captured, solution (A) of trimethylolaminomethane hydrochloride having a pH of 7.0, 2mM of mercaptoethanol and 5mM of EDTA was used to rinse for two bed volumes; and then solution (B) trimethylolaminomethane hydrochloride having a pH of 7.0, 2mM of mercaptoethanol, 5mM of EDTA and 1M of NaCl was used to form a gradient and elute the p43 protein captured in the column with 10 bed volumes, and protein absorption peak containing p43 protein was collected. The detection wavelength was 280nm. It was found that the p43 protein existed in the SP through-peak and the elution peak from electrophoresis result, with a ratio of 1:1 between the through peak and the elution peak. P43 protein was distributed widely such that it could not be effectively concentrated. Therefore, it is very difficult to separate and purify p43 protein.

### Example 3

The bacterial sludge of *Escherichia coli* expressing p43 protein obtained in example 1 was dissolved in a solution of 50mM of trimethylolaminomethane hydrochloride having a pH of 7.4, 2mM of mercaptoethanol and 5mM of EDTA at a ratio of 1:10. The cells were disrupted by high pressure homogenizing method. The disruption solution was centrifugated at 15000rpm and 4°C and the centrifugated deposit was removed. This solution was used as a loading solution, which was passed through an SP (GE Company) column. After captured, solution (A) of trimethylolaminomethane hydrochloride having a pH of 7.4, 2mM of mercaptoethanol and 5mM of EDTA was used to rinse for two bed volumes; and then solution (B) of trimethylolaminomethane hydrochloride having a pH of 7.4, 2mM of mercaptoethanol, 5mM of EDTA and 1M of NaCl was used to form a gradient and elute the p43 protein captured in the column with 10 bed volumes, and protein absorption peak containing p43 protein was collected. The detection wavelength was 280nm. It was found that the p43 protein existed in the SP through-peak and the elution peak from electrophoresis result, with a ratio of 2:1 between the through peak and the elution peak. P43 protein was distributed widely such that it could not be effectively concentrated. Therefore, it is very difficult to separate and purify p43 protein.

### Example 4

The bacterial sludge of *Escherichia coli* expressing p43 protein obtained in example 1 was dissolved in a solution of 50mM of trimethylolaminomethane hydrochloride having a pH of 7.0, 2mM of mercaptoethanol and 5mM of EDTA at a ratio of 1:10. The cells were disrupted by high pressure homogenizing method. The disruption solution was centrifugated at 15000rpm and 4°C and the centrifugated deposit was removed. This solution was used as a loading solution, which was passed through an DEAE Sepharose™ Fast Flow (GE Company) column. The balance solution and eluting solution were (A) a solution of trimethylolaminomethane hydrochloride having a pH of 7.0, 2mM of mercaptoethanol and 5mM of EDTA and (B) a solution of trimethylolaminomethane hydrochloride having a pH of 7.0, 2mM of mercaptoethanol, 5mM of EDTA and 1M of NaCl, respectively. The through-peak solution was collected. The through-peak solution collected from the DEAE ff column was used as a loading solution, and after diluting one time, passing it through the SP (GE Company) column. After captured, solution (A) of trimethylolaminomethane hydrochloride having a pH of 7.0, 2mM of mercaptoethanol and 5mM of EDTA was used to rinse the column for two bed volumes; and then solution (B) was used to form a gradient and elute p43 protein captured in the column with 10 bed volumes, and protein absorption peak containing p43 protein was collected. The detecting wavelength was 280nm. It was found that a small amount of p43 protein still existed in the SP through-peak, and the elution peak comprised about 50% of p43 protein from electrophoresis result.

### Example 5

The bacterial sludge of *Escherichia coli* expressing p43 protein obtained in example 1 was dissolved in a solution of 50mM of trimethylolaminomethane hydrochloride having a pH of 7.0, 2mM of mercaptoethanol and 5mM of EDTA at a ratio of 1:10. The cells were disrupted by high pressure homogenizing method. The disruption solution was centrifugated at 12000rpm and 4°C and the centrifugated deposit was removed to obtain a supernatant. The supernatant was salted out with 60% of ammonium sulfate to obtain a deposit. The deposit was dissolved in a solution of 50mM of trimethylolaminomethane hydrochloride having a pH of 7.0, 2mM of mercaptoethanol and 5mM of EDTA solution and desalted. The solution was passed through an SP (GE Company) column. After captured, solution (A) of trimethylolaminomethane hydrochloride having a pH of 7.0, 2mM of mercaptoethanol and 5mM of EDTA was used to rinse the column for two bed volumes; and then solution (B) of trimethylolaminomethane hydrochloride having a pH of 7.0, 2mM of mercaptoethanol, 5mM of EDTA and 1M of NaCl was used to form a gradient and elute p43 protein captured in the column with about 10 bed volumes, and protein absorption peak containing p43 protein was collected. The detecting wavelength was 280nm. It was found that a small amount of p43 protein still existed in the SP through-peak, and the elution peak comprised about 68% of p43 protein from electrophoresis result.

### Example 6

The bacterial sludge of *Escherichia coli* expressing p43 protein obtained in example 1 was dissolved in a solution of 50mM of trimethylolaminomethane hydrochloride having a pH of 7.8, 2mM of mercaptoethanol and 5mM of EDTA at a ratio of 1:10. The cells were disrupted by high pressure homogenizing method. The disruption solution was centrifugated at 15000rpm and 4°C and the centrifugated deposit was removed to obtain a supernatant. The supernatant was adjusted to pH of above 7.2 at 4°C. This solution was used as a loading solution, which was passed through an DEAE (GE Company) column. The balance solution and eluting solution were (A) a solution of trimethylolaminomethane hydrochloride having a pH of 7.6, 2mM of mercaptoethanol and 5mM of EDTA and (B) a solution of trimethylolaminomethane hydrochloride having a pH of 7.6, 2mM of mercaptoethanol, 5mM of EDTA and 1M of NaCl, respectively. The through-peak solution was collected. The through-peak solution collected from the DEAE ff column was used as a loading solution, and after diluting one time, passing it through the SP (GE Company) column. After captured, solution (A) of trimethylolaminomethane hydrochloride having a pH of 7.6, 2mM of mercaptoethanol and 5mM of EDTA was used to rinse the column for two bed volumes; and then solution (B) of trimethylolaminomethane hydrochloride having a pH of 7.6, 2mM of mercaptoethanol, 5mM of EDTA and 1M of NaCl was used to form a gradient and elute p43 protein captured in the column with about 10 bed volumes, and protein absorption peak containing p43 protein was collected. The detecting wavelength was 280nm. According to electrophoresis result, it was found that no p43 protein existed in the SP through-peak, and the elution peak comprised all of the p43 proteins. This showed that the p43 protein had uniform charge, therefore the effect from nucleic acid having negative charge was reduced. The p43 protein could effectively concentrate in the ion column according to property of the charge carried.

### Example 7

The bacterial sludge of *Escherichia coli* expressing p43 protein obtained in example 1 was dissolved in a solution of 50mM of trimethylolaminomethane hydrochloride having a pH of 7.8, 2mM of mercaptoethanol and 5mM of EDTA at a ratio of 1:10. The cells were disrupted by high pressure homogenizing method. The disruption solution was centrifugated at 15000rpm and 4°C and the centrifugated deposit was removed to obtain a supernatant. The supernatant was adjusted to pH of above 6.8 at 4°C. This solution was used as a loading solution, which was passed through an DEAE (GE Company) column. The balance solution and eluting solution were (A) a solution of trimethylolaminomethane hydrochloride having a pH of 7.0, 2mM of mercaptoethanol and 5mM of EDTA and (B) a solution of trimethylolaminomethane hydrochloride having a pH of 7.0, 2mM of mercaptoethanol, 5mM of EDTA and 1M of NaCl, respectively. The through-peak solution was collected. The through-peak solution collected from the DEAE ff column was used as a loading solution, and after diluting one time, passing it through the SP (GE Company) column. After captured, solution (A) of trimethylolaminomethane hydrochloride having a pH of 7.0, 2mM of mercaptoethanol and 5mM of EDTA was used to rinse the column for two bed volumes; and then solution (B) of trimethylolaminomethane hydrochloride having a pH of 7.0, 2mM of mercaptoethanol, 5mM of EDTA and 1M of NaCl was used to form a gradient and elute p43 protein captured in the column with about 10 bed volumes, and protein absorption peak containing p43 protein was collected. The detecting wavelength was 280nm. It was found that a small amount of p43 protein existed in the SP through-peak, most of which existed in the elution peak. And the elution peak comprised about 65% of p43 protein from electrophoresis result.

### Example 8

The bacterial sludge of *Escherichia coli* expressing p43 protein obtained in example 1 was dissolved in a solution of 50mM of trimethylolaminomethane hydrochloride having a pH of 8.0, 2mM of mercaptoethanol and 5mM of EDTA at a ratio of 1:10. The cells were disrupted by high pressure homogenizing method. The disruption solution was centrifugated at 15000rpm and 4°C and the centrifugated deposit was removed to obtain a supernatant. The supernatant was adjusted to pH of above 7.2 at 4°C. This solution was used as a loading solution, which was passed through an DEAE (GE Company) column. The balance solution and eluting solution were (A) a solution of trimethylolaminomethane hydrochloride having a pH of 7.4, 2mM of mercaptoethanol and 5mM of EDTA and (B) a solution of trimethylolaminomethane hydrochloride having a pH of 7.4, 2mM of mercaptoethanol, 5mM of EDTA and 1M of NaCl, respectively. The through-peak solution was collected. The through-peak solution collected from the DEAE ff column was used as a loading solution, and after diluting one time, passing it through the SP (GE Company) column. After captured, solution (A) of trimethylolaminomethane hydrochloride having a pH of 7.4, 2mM of mercaptoethanol and 5mM of EDTA was used to rinse the column for two bed volumes; and then solution (B) was used to form a gradient and elute the p43 protein captured in the column with about 10 bed volumes, and protein absorption peak containing p43 protein was collected. The detecting wavelength was 280nm. According to electrophoresis result, it was found that no p43 protein existed in the SP through-peak, and the elution peak comprised all of the p43 proteins. This showed that the p43 protein had uniform charge, therefore the effect from nucleic acid having negative charge was reduced. The p43 protein could effectively concentrate in the ion column according to property of the charge carried.

### Example 9

The bacterial sludge of *Escherichia coli* expressing p43 protein obtained in example 1 was dissolved in a solution of 20mM of phosphate solution having a pH of 7.8, 2mM of mercaptoethanol and 5mM of EDTA at a ratio of 1:10. The cells were disrupted by high pressure homogenizing method. The disruption solution was centrifugated at 15000rpm and 4 °C and the centrifugated deposit was removed to obtain a supernatant. The supernatant was adjusted to pH of above 7.2 at 4°C. This solution was used as a loading solution, which was passed through an DEAE (GE Company) column. The balance solution and eluting solution were (A) a solution of 20mM phosphate solution having a pH of 7.4, 2mM of mercaptoethanol and 5mM of EDTA and (B) a solution of 20mM phosphate solution having a pH of 7.4, 2mM of mercaptoethanol, 5mM of EDTA and 1M of NaCl, respectively. The through-peak solution was collected. The through-peak solution collected from the DEAE ff column was used as a loading solution, and after diluting one time, passing it through the SP (GE Company) column. After captured, solution (A) of 20mM phosphate solution having a pH of 7.4, 2mM of mercaptoethanol and 5mM of EDTA was used to rinse the column for two bed volumes; and then solution (B) of trimethylolaminomethane hydrochloride having a pH of 7.4, 2mM of mercaptoethanol, 5mM of EDTA and 1M of NaCl was used to form a gradient and elute p43 protein captured in the column with about 10 bed volumes, and protein absorption peak containing p43 protein was collected. The detecting wavelength was 280nm. According to the electrophoresis result, it was found that a small amount of p43 protein existed in the SP through-peak, most of which, in a ratio of about 86%, existed in the elution peak.

### Example 10

The bacterial sludge of *Escherichia coli* expressing p43 protein obtained in example 1 was dissolved in a solution of 100mM of phosphate solution having a pH of 7.8, 2mM of mercaptoethanol and 5mM of EDTA at a ratio of 1:10. The cells were disrupted by high pressure homogenizing method. The disruption solution was centrifugated at 15000rpm and 4 °C and the centrifugated deposit was removed to obtain a supernatant. The supernatant was adjusted to pH of above 7.2 at 4°C. This solution was used as a loading solution, which was passed through an DEAE (GE Company) column. The balance solution and eluting solution were (A) a solution of 100mM phosphate solution having a pH of 7.4, 2mM of mercaptoethanol and 5mM of EDTA and (B) a solution of 100mM phosphate solution having a pH of 7.4, 2mM of mercaptoethanol, 5mM of EDTA and 1M of NaCl, respectively. The through-peak solution was collected. The through-peak solution collected from the DEAE ff column was used as a loading solution, and after diluting one time, passing it through the SP (GE Company) column. After captured, solution (A) of 100mM phosphate solution having a pH of 7.4, 2mM of mercaptoethanol and 5mM of EDTA was used to rinse the column for two bed volumes; and then solution (B) was used to form a gradient and elute p43 protein captured in the column with about 10 bed volumes, and protein absorption peak containing p43 protein was collected. The detecting wavelength was 280nm. According to electrophoresis result, it was found that no p43 protein existed in the SP through-peak, and the elution peak comprised all of p43 proteins.

## Claims

1. A process of producing human recombinant p43 protein from genetic engineering *Escherichia coli* containing p43 protein gene, comprising the following steps:
(1) suspending genetic engineering *Escherichia coli* containing p43 protein gene by buffer solution to obtain a suspension;
(2) disrupting and centrifuging the suspension obtained in step (1) to obtain a supernatant;
(3) salting out the supernatant obtained in step (2) with ammonium sulfate solution to obtain a precipitate, and dissolving the precipitate with a buffer solution to obtain a solution;
(4) passing the solution obtained in step (3) through an SP Sepharose™ Fast Flow column;
(5) eluting the SP Sepharose™ Fast Flow column with a buffer solution to obtain an p43 protein solution.

2. The process according to claim 1, wherein the buffer solution is one or more solution selected from the group consisting of trimethylolaminomethane hydrochloride and phosphate solutions.

3. The process according to claim 1, wherein the buffer solution has a pH of from 6.5 to 9.0.

4. The process according to claim 1, wherein the buffer solution has a pH of from 7 to 8.5.

5. The process according to claim 1, wherein the buffer solution has a pH of from 7.0 to 8.0.

## Patentansprüche

1. Verfahren zur Herstellung von humanem rekombinanten p43-Protein aus gentechnischem *Escherichia coli,* das das Gen für p43-Protein aufweist, das die folgenden Schritte umfasst:
(1) Suspendieren des gentechnischen *Escherichia coli,* das das Gen für p43-Protein aufweist, mit Hilfe einer Pufferlösung zur Bildung einer Suspension;
(2) Zerstören und Zentrifugieren der in Schritt (1) hergestellten Suspension zur Bildung eines Überstands;
(3) Aussalzen des in Schritt (2) hergestellten Überstands mit einer Ammoniumsulfatlösung zur Bildung eines Niederschlags und Auflösen des Niederschlags mit Hilfe einer Pufferlösung zur Bildung einer Lösung;
(4) Hindurchlaufenlassen der in Schritt (3) hergestellten Lösung durch eine SP Sepharose^{™}-Säule mit schneller Fließge-schwindigkeit;
(5) Eluieren der SP Sepharose^{™}-Säule mit schneller Fließge-schwindigkeit mit einer Pufferlösung, um die p43-Proteinlösung zu erhalten.

2. Verfahren nach Anspruch 1, worin es sich bei der Pufferlösung um eine oder mehrere Lösungen handelt, die unter Trimethylol-aminomethan-Hydrochlorid und Phosphatlösungen ausgewählt sind.

3. Verfahren nach Anspruch 1, worin die Pufferlösung einen pH-Wert von 6,5 bis 9 aufweist.

4. Verfahren nach Anspruch 1, worin die Pufferlösung einen pH-Wert von 7 bis 8,5 aufweist.

5. Verfahren nach Anspruch 1, worin die Pufferlösung einen pH-Wert von 7,0 bis 8,0 aufweist.

## Revendications

1. Procédé pour la production de la protéine p43 humaine recombinante par une *Escherichia coli* à modification génétique contenant le gène de la protéine p43, comprenant les étapes suivantes :
(1) mise en suspension d'une *Escherichia coli* à modification génétique contenant le gène de la protéine p43 au moyen d'une solution tampon pour obtenir une suspension ;
(2) dissociation et centrifugation de la suspension obtenue dans l'étape (1) pour obtenir un surnageant ;
(3) relargage du surnageant obtenu dans l'étape (2) avec une solution de sulfate d'ammonium pour obtenir un précipité, et dissolution du précipité avec une solution tampon pour obtenir une solution ;
(4) passage de la solution obtenue dans l'étape (3) à travers une colonne SP Sepharose™ Fast Flow ;
(5) élution de la colonne SP Sepharose™ Fast Flow avec une solution tampon pour obtenir une solution de protéine p43.

2. Procédé suivant la revendication 1, dans lequel la solution tampon est une ou plusieurs solutions choisies dans le groupe consistant en des solutions de chlorhydrate et de phosphate de triméthylolaminométhane.

3. Procédé suivant la revendication 1, dans lequel la solution tampon a un pH de 6,5 à 9,0.

4. Procédé suivant la revendication 1, dans lequel la solution tampon a un pH de 7 à 8,5.

5. Procédé suivant la revendication 1, dans lequel la solution tampon a un pH de 7,0 à 8,0.
